# EUROPEAN PATENT APPLICATION

(11) **EP 4 461 798 A1**
(43) Date of publication of application: **13.11.2024**
(21) Application number: 23461576.3
(22) Date of filing: 07.05.2023
(51) Int. Cl.: C12G 1/02, C12N 1/18

(54) **MEDIUM FROM DRIED CRICKETS GRYLLUS CAMPESTRIS FOR YEAST, METHOD OF PRODUCTION THEREOF AND ITS USE AS A MEDIUM FOR WINE YEAST**

(71) Applicant: Uniwersytet Zielonogórski, 65-417 Zielona Góra (PL)
(72) Inventor: Kliks, Jaroslaw, 66-120 Kargowa (PL); Korycka-Korwek, Justyna, 62-128 Zielona Gora (PL); Mrowczynska, Maria, 62-273 Zielona Gora (PL); Krolicka, Zuzanna, 66-100 Sulechow (PL); Czabaj, Slawomir, 21-110 Ostrow Lubelski (PL)
(74) Representative: JD&P Patent Attorneys Joanna Dargiewicz & Partners

(57) **Abstract**

The subject of the present invention is a method for producing a medium solution from dried field crickets *Gryllus campestris* for yeast, characterized in that includes steps where: shredded dried field crickets *Gryllus campestris* is mixed with water in a ratio of 1:2.5 to 1:7.5 and the resulting mixture is heated to 40°C; an enzyme mixture comprising alpha amylase, beta amylase, pepsin and elastase is added and stirred with heating to 40°C for 12-36 hours, preferably for 24 hours; the precipitates obtained are separated and the filtrate is concentrated to a dry matter content of 25% to 35% by weight, preferably 30% by weight; concentrated filtrate constituting a protein concentrate is mixed with magnesium sulphate (VI), potassium dihydrogen phosphate (V), zinc chloride and glucose in the proportion of protein concentrate 93.00-95.00%, magnesium sulphate (VI) 0.40-0.60%, potassium dihydrogen phosphate (V) 0.40-0.60%, zinc chloride 0.15-0.30%, glucose 4.50-5.00% to obtain yeast medium. The present invention also relates to the medium obtained by this method and its use.

## Description

The subject of the present invention is a medium solution from dried crickets *Gryllus campestris* for yeast, a method for producing such a yeast medium, and the use of such a medium as a wine yeast medium.

The proper course of the fermentation process depends not only on the sugar content in the fermentation medium, but also on the physiological state of the yeast and the availability of mediums. Yeast cells need zinc and magnesium ions and a source of nitrogen to function properly.

Many researchers have shown a positive effect of supplementation with protein hydrolysates on the dynamics and final effects of alcoholic fermentation processes. Proper preparation of the yeast mass consists in cell rehydration and proper nutrition. In the first adaptive phase of the fermentation process, the yeast cells multiply. Preferably, the fermentation takes place in a highly oxygenated environment. In the multiplication phase, it is important that there is a high concentration of easily digestible protein with a high content of essential amino acids in the environment.

Classic wine mediums are mixtures of mineral salts that meet the needs of yeast cells for sodium, potassium, magnesium, zinc, phosphorus and even chromium. One of the most commonly used sources of protein is the so-called yeast hydrolyzate, which provides, in addition to the complex of minerals, also amino acids and peptides that are components of yeast cells.

Korean patent KR.101888921B1 discloses a method for producing a seasoning ingredient having remarkably reduced processing time and showing excellent sensitivity even when soybean is replaced by insects, by including the steps of: sequentially fermenting insects with *Rhizopus oligosporus* and *Bacillus subtilis* to obtain the first fermented product; fermenting the soybean with *Aspergillus oryzae* to obtain a second fermented product; and aging the first fermented product with the second fermented product and the brine. *Rhizopus oligosporus* insects have been isolated and *Bacillus subtilis,* to get the first fermented product; fermenting soybeans with *Aspergillus oryzae* to make a second fermented product; and mixing and aging the first fermented product with the second fermented product and the brine. The insect is selected from the group consisting of a brown duck larva, a pair of crickets, a silkworm larva, a long-neck beetle larva, a white mackerel.

Chinese patent application CN107624953A discloses a method for preparing a selenium-rich high-protein active peptide supplement. The method includes: subjecting feed grade sodium selenite and dry yeast powder solution to fermentation reaction to prepare organic selenium liquid yeast, carrying out mixed fermentation on organic selenium yeast, fermented chicken manure, feather meal, meat and bone meal, egg shell, enzyme preparation, molasses and the like, controlling the water content of the resulting fermented material to about 70%, and cultivating the housefly larvae for a period of time. Then the housefly larvae are extracted and washing is carried out, after which the washed housefly larvae and liquid selenium yeast are mixed fermentation for 24 hours to get selenium-rich active peptide extraction liquid with high protein content, feed grade calcium powder is used for adsorption, a heat pump is used for drying, and grinding to 300-800 mesh diameter is performed, thereby obtaining a selenium-rich additive raw material for a high protein active peptide.

Chinese patent application CN108975970A discloses a method for producing organic granular fertilizer by using mushroom paste from silkworm droppings. The production method includes the following steps: mixing and fermenting deodorizing bacteria, silkworm excrement, paste of edible mushrooms and apple bark, and deodorizing; adding seaweed meal, dioscoreae rhizome powder, cricket powder, mussel powder, shrimp head powder, oil, bagasse and a complex biological bacterium agent to the deodorized fermentation product; after evenly mixing, sealing and fermenting: adding nano sepiolite, activated phosphate ore powder, plant amino acid powder, nano diatomite, high-molecular waterabsorbing resin, urea, medium and trace elements, ammonium dihydrogen phosphate, potassium sulfate, manganese sulfate, calcium nitrate and magnesium sulfate and mixing to obtain a mixture; sterilizing the mixture and carrying out a drying treatment; followed by crushing and screening.

The aim of the present invention was to develop a method for obtaining a high-protein, peptide- and amino acid-rich medium for yeast, especially for wine yeast, using field crickets as a medium substrate.

This has been achieved by the subject of the present invention.

Thus, the present invention relates to a method for producing a medium solution from dried field crickets *Gryllus campestris* for yeast, characterized in that includes steps where: shredded dried field crickets *Gryllus campestris* is mixed with water in a ratio of 1:2.5 to 1:7.5 and the resulting mixture is heated to 40°C; an enzyme mixture comprising alpha amylase, beta amylase, pepsin and elastase is added and stirred with heating to 40°C for 12-36 hours, preferably for 24 hours; the precipitates obtained are separated and the filtrate is concentrated to a dry matter content of 25% to 35% by weight, preferably 30% by weight; concentrated filtrate constituting a protein concentrate is mixed with magnesium sulphate (VI), potassium dihydrogen phosphate (V), zinc chloride and glucose in the proportion of protein concentrate 93.00-95.00%, magnesium sulphate (VI) 0.40-0.60%, potassium dihydrogen phosphate (V) 0.40-0.60%, zinc chloride 0.15-0.30%, glucose 4.50-5.00% to obtain yeast medium. The present invention also relates to the medium obtained by this method and its use.

According to the invention, the method of obtaining of amino acids and peptides from *Gryllus campestris* field crickets has been developed, in the form of a concentrated and membrane-purified preparation. This preparation has a beneficial effect on yeast cells, increasing their resistance to high concentrations of sugars and ethyl alcohol.

### EXAMPLES

### Example 1 - preparation method

10 kg of dried crickets *Gryllus campestris* was ground in a hammer mill and transferred to a boiler equipped with a heating jacket and a stirrer. 50 kg of water was added to the kettle and the mixture was heated to 40°C. Then 180 g of alpha amylase, 220 g of beta amylase, 150 g of pepsin and 100 g of elastase were added. Stirring at 100 RPM and a temperature of 40°C was maintained for 24 hours. After this time, the mixture was centrifuged in a centrifuge to separate the precipitates. The rotation of the centrifuge was 4000 rpm, the flow through the centrifuge was 200 dm³/h. The centrifuged fluid was subjected to microfiltration on a ceramic membrane with a pore diameter of 0.14 µm, using a circulation flow of 8000 dm³/h, transmembrane pressure of 50 000 Pa, retentate flow of 400 dm³/h, permeate flow of 40 dm³/h.

The obtained filtrate (permeate) was concentrated on an ultrafiltration system using a polymer membrane with a pore size of 4 kDa. Using a circulation flow of 10000 dm³/h, transmembrane pressure of 100 000 Pa, retentate flow of 200 dm³/h, permeate flow of 60 dm³/h. Membrane densification was carried out to a dry matter level of 30 wt%.

The concentrated preparation was transferred to a mixer in which it was combined with the other components of the medium according to the recipe presented in Table 1.

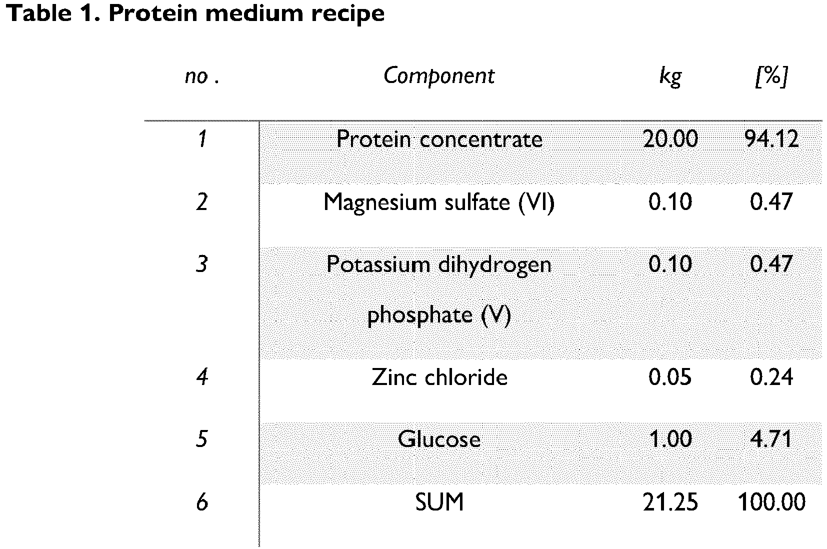

The solution was stirred for 1 hour in a mixer equipped with a 130 rpm helical stirrer.

After this time, the medium was poured into trays and subjected to freezing at -70°C, followed by lyophilization according to the following program:
preliminary drying - 2000 Pa, tray temperature 28°C, time 24 h,
drying - 200 Pa, tray temperature 35°C, time 24 h.
After drying, the powder was ground in a ball mill and vacuum packed in PA/PE packaging.

### Example 2 - use of the preparation as a medium for wine yeast

The formulation prepared as described in Example 1 served as a starter medium for wine yeast. The yeast was rehydrated as follows: 50 g of *Saccharommyces cerevisiae* yeast were added to 2 dm³ of distilled water at 25°C and 20 g of medium. The solution was stirred (100 rpm) and allowed to incubate for 4 hours.

Cream yeast prepared as described above was used as a biological material in fermentation tests. In the same trials, reference material was also used, which was 50 g of *Saccharommyces cerevisiae* yeast rehydrated in 2 dm³ of distilled water with the addition of yeast hydrolyzate extract.

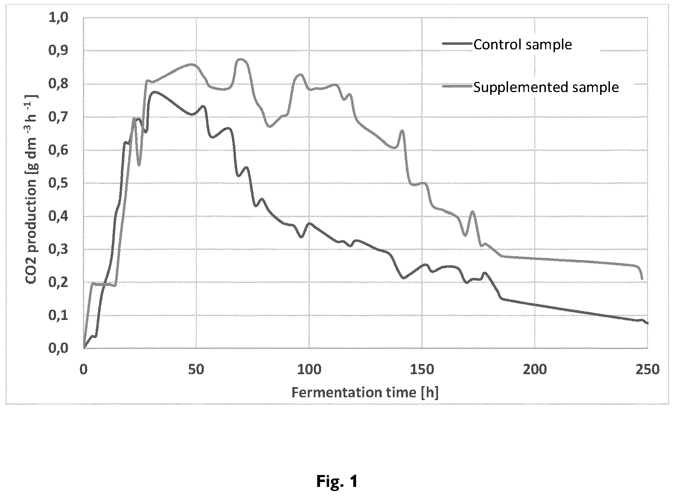

125 l of grape must containing 243 g/dm³ of simple sugars were used for fermentation tests. Fig. 1 shows the fermentation dynamics defined as the amount of CO₂ produced with 1 dm³ of must during 1 hour of fermentation. In the sample supplemented with the preparation of hydrolyzed crickets, a much higher dynamics of CO ₂ production was noted throughout the turbulent fermentation. In the case of the supplemented batch and the control sample, convergent dynamics could be observed up to 27 hours of the process. In the following hours of fermentation, the supplemented solution was characterized by significantly higher fermentation dynamics.

Significant differences were also observed in the final effects of the fermentation shown in Table 2. The final effects were assessed at 250 hours of the process. The collected samples were subjected to HPLC analysis in order to determine the content of alcohol and monosaccharides, and the diameter of yeast cells was also assessed microscopically. As a result of the analysis, a much higher degree of attenuation was shown in the samples supplemented with a medium prepared on the basis of crickets. A significantly higher alcohol content was found, 106.92 g/dm^{3,} as well as a lower concentration of residual sugars, 15.2 g/dm^{3.} The larger average diameter of yeast cells may mean that the supplemented cells are much more resistant to high concentrations of ethyl alcohol.

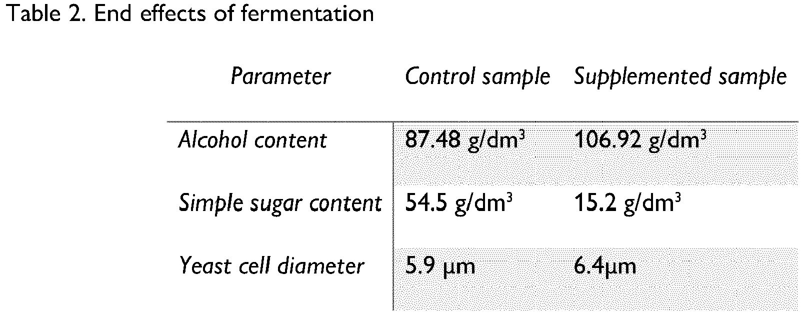

## Claims

1. A method for producing a medium from dried field crickets *Gryllus campestris* for yeast, **characterized in that** it comprises the steps in which:
- shredded dried field crickets *Gryllus campestris* are mixed with water in a ratio of 1:2.5 to 1:7.5 and the resulting mixture is heated to 40°C;
- an enzyme mixture comprising alpha amylase, beta amylase, pepsin and elastase is added and stirred with heating to 40°C for 12-36 hours, preferably for 24 hours;
- the precipitates obtained are separated and the filtrate is concentrated to a dry matter content of 25% to 35% by weight, preferably 30% by weight;
- the concentrated filtrate, which is a protein concentrate, is mixed with magnesium sulphate (VI), potassium dihydrogen phosphate (V), zinc chloride and glucose in the following proportion:
protein concentrate 93.00-95.00%,
magnesium sulfate (VI) 0.40-0.60%,
potassium dihydrogen phosphate (V) 0.40-0.60%,
zinc chloride 0.15-0.30%,
glucose 4.50-5.00%,
to obtain the yeast medium.

2. The method of claim 1, **characterized in that** the obtained medium is then subjected to lyophilization and the resulting powder is crushed.

3. The method of claim 1 or 2, **characterized in that** the crushed dried field crickets *Gryllus campestris* is mixed with water in a ratio of 1:5.

4. The method of claim 1, 2 or 3, **characterized in that** the weight ratio of the aqueous mixture of dried field crickets *Gryllus campestris* to the enzyme mixture ranges from 95:1 to 100:1.

5. The method according to any one of claims 1-4, **characterized in that** 10 kg of crickets, 50 kg of water, 650 g of an enzyme mixture containing, 180 g of alpha amylase, 220 g of beta amylase, 150 g of pepsin and 100 g of elastase are used.

6. The method according to any one of claims 1-5, **characterized in that** the concentrated filtrate constituting a protein concentrate is mixed with magnesium sulphate, potassium dihydrogen phosphate, zinc chloride and glucose in the proportion:

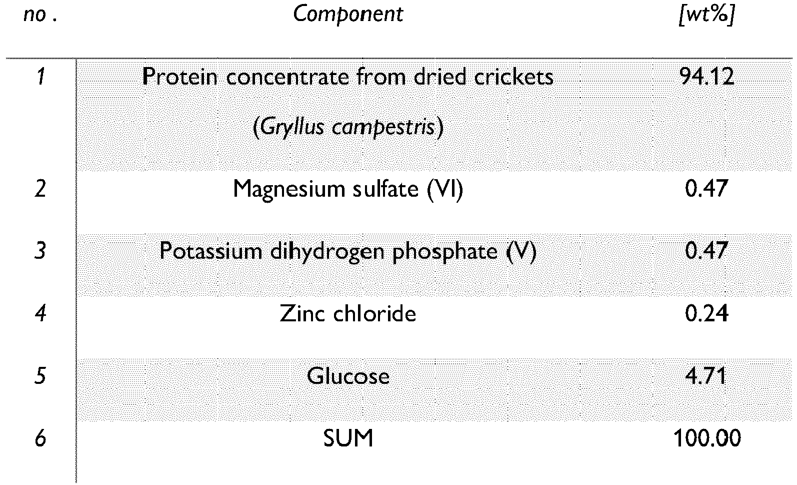

7. A medium for yeast from dried field crickets *Gryllus campestris* comprising the following ingredients in proportion:

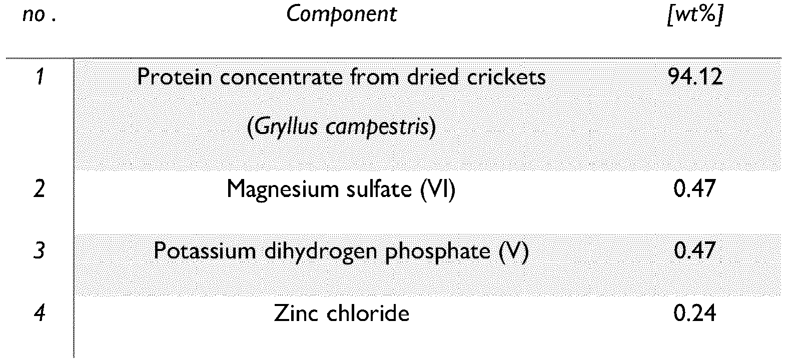

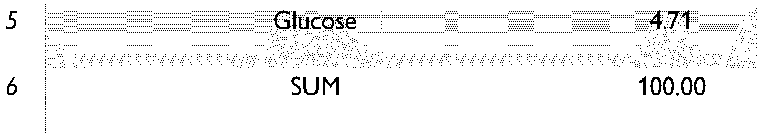

8. The medium for yeast according to claim 7 obtained by a method as defined in any one of claims 1-6.

9. A use of the medium according to claim 7 or 8 as a medium for yeast, preferably for wine yeast.
